# EUROPÄISCHE PATENTSCHRIFT

(11) **EP 2 997 540 B1**
(45) Veröffentlichungstag und Bekanntmachung des Hinweises auf die Patenterteilung: **22.12.2021**
(21) Anmeldenummer: 14726120.0
(22) Anmeldetag: 16.05.2014
(51) Int. Cl.: G06T 5/00, G06T 7/00, G06T 7/73, A61B 6/00

(54) **INTRAOPERATIVE BILDREGISTRIERUNG MITTELS BEZUGSMARKERN**
INTRAOPERATIVE IMAGE REGISTRATION USING FIDUCIAL MARKS
RECALAGE D'IMAGES INTRAOPERATIVES UTILISANT DES MARQUES DE REPÈRE

(30) Priorität: 16.05.2013 DE 102013209158
(43) Veröffentlichungstag der Anmeldung: 23.03.2016
(73) Patentinhaber: Intersect ENT GmbH, 16761 Hennigsdorf (DE)
(72) Erfinder: KRÜGER, Timo, 13469 Berlin (DE); MUCHA, Dirk, 13467 Berlin (DE)
(74) Vertreter: Eisenführ Speiser
(86) Internationale Anmeldenummer: PCT/EP2014/060172
(87) Internationale Veröffentlichungsnummer: WO 2014/184382

(56) Entgegenhaltungen:
- US-A- 6 118 845
- US-A1- 2003 130 576
- US-A1- 2005 281 385
- MAINTZ J B A ET AL: "A SURVEY OF MEDICAL IMAGE REGISTRATION", MEDICAL IMAGE ANALYSIS, OXFORDUNIVERSITY PRESS, OXFORD, GB, Bd. 2, Nr. 1, 1. Januar 1998 (1998-01-01), Seiten 1-37, XP001032679, ISSN: 1361-8423, DOI: 10.1016/S1361-8415(01)80026-8
- CALVIN R MAURERJR ET AL: "Registration of Head Volume Images Using Implantable Fiducial Markers", IEEE TRANSACTIONS ON MEDICAL IMAGING, IEEE SERVICE CENTER, PISCATAWAY, NJ, US, Bd. 16, Nr. 4, 1. August 1997 (1997-08-01) , XP011035646, ISSN: 0278-0062
- DANG H ET AL: "Robust methods for automatic image-to-world registration in cone-beam CT interventional guidance", MEDICAL PHYSICS, AIP, MELVILLE, NY, US, vol. 39, no. 10, 1 October 2012 (2012-10-01), pages 6484-6498, XP012160677, ISSN: 0094-2405, DOI: 10.1118/1.4754589 [retrieved on 2012-10-03]
- R. Hofstetter ET AL: "Fluoroscopy as an Imaging Means for Computer-Assisted Surgical Navigation", COMPUTER AIDED SURGERY, vol. 4, no. 2, 1 January 1999 (1999-01-01) , pages 65-76, XP055683105, US ISSN: 1092-9088, DOI: 10.3109/10929089909148161

## Beschreibung

Die Erfindung betrifft ein Verfahren zum Einbinden von mittels eines bildgebenden Verfahrens, insbesondere eines tomografischen Verfahrens gewonnener Bilddaten in ein System zur Operationsplanung und/oder zur intraoperativen Navigation, wobei die Bilddaten zunächst oder zuvor mittels bildgebender Verfahren wie Röntgen und/oder Computertomografie gewonnen werden oder wurden. Die Erfindung betrifft auch einen Referenzkörper, einen elektromagnetischen Feldgenerator, sowie ein medizinisches System, jeweils insbesondere zur Verwendung in einem derartigen Verfahren.

Die Verwendung von präoperativ gewonnenen, i.d.R. in digitalisierter Form vorliegenden Bilddaten eines Patienten, z.B. Röntgen, oder Computer-, oder Kernspintomografieaufnahmen für die Planung und Durchführung invasiver chirurgischer Eingriffe ist grundsätzlich bekannt. Derartige Verfahren liefern "Innenansichten" von Organen oder Körperteilen.

In einigen Verfahren werden diese Bilddaten auch während der Operation erstellt bzw. aktualisiert, um etwaige Veränderungen am aufgenommenen Objekt, z.B. den Operationsfortschritt oder Reaktionen des Körpers auf den chirurgischen Eingriff, die nicht oder nur schlecht von Film- oder Videokameras erfassbar sind, zu visualisieren. Aus tomografisch gewonnenen Bilddaten können dreidimensionale Modelle eines oder mehrerer Körperteile und/oder Organe errechnet werden.

Darüber hinaus ist es üblich, optische Aufnahmen wie Fotografien oder Videobilder des Patienten intraoperativ, z.B. mit einem Endoskop aufzunehmen und zusammen mit den präoperativ erstellten, tomografischen Bilddaten auf einem Monitor jeweils als Einzelbild oder überlagert darzustellen. Auf diese Weise können beispielsweise zu entfernendes Gewebe oder im Operationsgebiet liegende und durch den chirurgischen Eingriff potenziell gefährdete Nervenbahnen oder Gefäße dem Chirurgen besser sichtbar gemacht werden. Diese Darstellung der Bilddaten ermöglicht es dem Chirurgen, die bei der Operation verwendeten medizinischen Geräte möglichst effizient und unter minimaler Beeinträchtigung des umliegenden Gewebes des Patienten einzusetzen.

Computertomografisch gewonnene Bilddaten (im Folgenden als tomografische Bilddaten bezeichnet) oder röntgentechnisch gewonnene Bilddaten ermöglichen das Visualisieren von Bereichen im menschlichen Körper, die für das Erstellen von fotografisch oder videotechnisch gewonnenen Bilddaten (im Folgenden als F-/V-Bilddaten bezeichnet) ein invasives Verfahren erfordern würden. Dafür haben tomografische Bilddaten gegenüber F-/V-Bilddaten den Nachteil, dass sie i.d.R. die aufgenommenen Bereiche nicht maßstabsgetreu sondern verzerrt darstellen. Die Verzerrung der tomografischen Bilddaten ist systembedingt und unterliegt keiner vorhersehbaren Gesetzmäßigkeit, nach der sie durch vorgegebene Korrekturalgorithmen korrigiert werden könnte. Eine Einbindung von tomografischen Bilddaten in ein System zur Operationsplanung und/oder intraoperativen Navigation, wie z.B. einem Lageerfassungssystem, erfordert somit eine möglichst fehlerfreie Entzerrung der tomografischen Bilddaten, um eine gewünschte Genauigkeit zu erreichen.

Bekannte Entzerrungsverfahren vergleichen die verzerrten tomografischen Bilddaten mit entsprechenden F-/V-Bilddaten, die denselben Bereich, vorzugsweise aus derselben Perspektive abbilden, und führen aufgrund der ermittelten Abweichungen der tomografischen Bilddaten von den F-/V-Bilddaten eine Entzerrung der tomografischen Bilddaten durch. Anschließend können die Bilddaten z.B. überlagert auf einer Anzeige dargestellt werden.

Derartige bekannte Verfahren zum Entzerren von tomografischen Bilddaten haben den Nachteil, dass sie oftmals fehlerhafte Ergebnisse produzieren. Ein Grund hierfür ist, dass eine gleiche Perspektive bei der Erstellung der tomografischen Bilddaten und F-/V-Bilddaten nicht genau erreichbar ist und somit eine Entzerrung anhand perspektivisch unterschiedlicher F-/V-Bilddaten erfolgt. Ferner werden die unterschiedlichen Bilddaten oftmals zu unterschiedlichen Zeitpunkten erstellt, so dass z.B. akute Schwellungen, Hämatome etc. zu Fehlern bei der Entzerrung der tomografischen Bilddaten führen können.

Aus dem Stand der Technik sind auch Verfahren bekannt, welche die Verzerrungen eines einzelnen Aufnahmesystems durch eine vorherige oder wiederholte Kamerakalibrierung ausgleichen, wobei ein dem jeweiligen Aufnahmesystem angepasster bekannter Referenzkörper für dessen Kalibrierung verwendet wird, vgl. z.B. US 2003/130576 A1.

Der Erfindung liegt die Aufgabe zugrunde, ein zuverlässigeres Verfahren zur Einbindung von tomografischen Bilddaten in ein System zur Operationsplanung bzw. zur intraoperativen Navigation anzugeben. Der Erfindung liegt ebenfalls die Aufgabe zugrunde einen Referenzkörper, einen elektromagnetischen Feldgenerator, sowie ein medizinisches System bereitzustellen, die jeweils zur Verwendung in einem derartigen Verfahren geeignet sind.

Erfindungsgemäß wird die Aufgabe gelöst durch die in den Ansprüchen 1-14 definierte Lösung. Grob gesprochen betrifft die Erfindung ein Verfahren zur Einbindung von computertomografisch und/oder röntgentechnisch gewonnenen Bilddaten in ein System zur Operationsplanung und/oder zur intraoperativen Navigation, welches die folgenden Schritte aufweist:
- Aufnehmen von tomografischen Bilddaten oder röntgentechnisch gewonnenen Bilddaten von zumindest einem definierten Körperbereich des Patienten mittels mindestens einem hierfür geeigneten ersten Aufnahmegerät, wobei ein erster Referenzkörper mit mindestens einer Oberfläche an dem Patienten angeordnet ist und von dem ersten Aufnahmegerät mit aufgenommen wird;
- Vergleichen der den ersten Referenzkörper repräsentierenden aufgenommen Bilddaten mit bekannten geometrischen Daten des ersten Referenzkörpers zum Gewinnen von Verzerrungsinformation
- Entzerren der aufgenommen Bilddaten durch eine Recheneinheit auf Basis der Verzerrungsinformation zum Gewinnen entzerrter Bilddaten;
- Überlagern der entzerrten Bilddaten mit weiteren Bilddaten desselben Körperbereichs des Patienten zum Gewinnen überlagerter Bilddaten; und
- Darstellen der überlagerten Bilddaten auf einer Anzeige.

Das Verfahren schließt den Gedanken ein, präoperativ eine Computertomografie anzufertigen und intraoperativ Röntgenbilder (Fluoroskopiebilder) mit einem C-Bogen anzufertigen. Durch den Körper mit bekannter Geometrie können die intraoperativ aufgenommenen Röntgenbilder (Fluoroskopiebilder) entzerrt werden und so mit virtuell, aus dem präoperativ aufgenommenen, beispielsweise von einem Computertomografen generierten Röntgenbildern verglichen werden. Dies hat den Vorteil, dass sich ein Arzt bei der Operationsplanung und bei der Durchführung der Operation an Röntgenbildern orientieren kann, und zwar sowohl präoperativ aufgenommenen wie intraoperativ aufgenommenen Röntgenbildern. Anstelle von röntgentechnisch gewonnenen Bilddaten können Bilddaten auch mit einem anderen bildgebenden Verfahren, z.B. einem Ultraschallbasiertem Verfahren aufgenommen werden. Genauso können die tomografischen Bilddaten mittels eines Computertomografen, eines Magnet-Resonanz-Tomografen, eines Ultraschall-Tomografen oder dergleichen gewonnen werden.

Vorzugsweise liegen die geometrischen Daten des ersten Referenzkörpers als Bilddaten vor, die die tatsächliche Geometrie des ersten Referenzkörpers unverzerrt abbilden.

Vorzugsweise umfassen die weiteren Bilddaten desselben Körperbereichs des Patienten die geometrischen Daten des ersten Referenzkörpers als Bilddaten.

Weiter ist es bevorzugt, wenn der erste Referenzkörper bei der Aufnahme der weiteren Bilddaten die gleiche Lage zum Aufnahmegerät und/oder Patienten aufweist wie bei der Aufnahme der tomografischen Bilddaten, um beim Entzerren der Bilddaten nicht unterschiedliche Lagen berücksichtigen zu müssen.

Auch ist es vorteilhaft, wenn der erste Referenzkörper bei der Aufnahme der weiteren Bilddaten den gleichen Abstand zum Aufnahmegerät aufweist, wie bei der Aufnahme der tomografischen Bilddaten, so dass nach Möglichkeit keine unterschiedlichen Verzerrungen auftreten.

Vorteilhaft ist es auch, wenn der erste Referenzkörper während des Erstellens der tomografischen Bilddaten derart ausgerichtet ist, dass der größtmögliche Teil der Oberfläche des ersten Referenzkörpers dem Aufnahmegerät zugewandt ist, um auf diese Weise möglichst signifikante Bilddaten vom Referenzkörper aufnehmen zu können.

Vorzugsweise ist der erste Referenzkörper an einer Körperstelle des Patienten angeordnet, so dass die Körperstelle und der erste Referenzkörper sich mit einer möglichst detaillierten Aufnahme erfassen lassen.

Die weiteren Bilddaten desselben Körperbereichs des Patienten können von einem zweiten Aufnahmegerät, vorzugsweise einem Röntgengerät erstellt werden und ein Röntgenbild bzw. Fluoroskopiebilder umfassen.

Vorzugsweise weist der Referenzkörper Lokalisatoren, wie z.B. Sensorspulen oder optische Lokalisatoren auf. Dies erlaubt es, nicht nur die aufgenommen Bilddaten mit bekannten geometrischen Daten auf dem Wege der Entzerrung miteinander abzugleichen, sondern außerdem auch das Referenzfeld für die intraoperative Navigation. Dies kann im Falle der an sich bekannten elektromagnetischen Navigation ein von einem Feldgenerator erzeugtes Wechselfeld sein, welches von den Sensorspulen erfasst wird. Ein derartiger Abgleich der aufgenommen Bilddaten mit den bekannten geometrischen Daten und dem Referenzfeld für die intraoperative Navigation erlaubt eine Registrierung, die nicgt nur präoperativ, sondern auch intraoperativ durchgeführt werden kann.

Der erste Referenzkörper ist vorzugsweise ringförmig oder kreisförmig und/oder weist ringförmige und/oder kreisförmige Bereiche und/oder Elemente auf.

Vorzugsweise werden die weiteren Bilddaten intraoperativ aufgenommen und sind somit höchst aktuell.

Die Anzeige umfasst vorzugsweise einen Monitor.

Der definierte Körperbereich des Patienten umfasst vorzugsweise die Wirbelsäule, d.h. das Verfahren ist insbesondere für die Unterstützung von Untersuchungen oder Behandlungen im Bereich der Wirbelsäule vorteilhaft.

Zumindest ein Teil der tomografischen Bilddaten kann vor einer Untersuchung oder Operation aufgenommen werden, z.B. in einem Tomografen, etwa einem Kernspintomografen, der die Untersuchung oder Operation ansonsten behindern würde.

Besonders bevorzugt ist ein zweiter oder weiterer Referenzkörper an einem Operationsort im Körper des Patienten angeordnet, wobei der Operationsort im definierten Körperbereich des Patienten angeordnet ist. Der erste Referenzkörper und der zweite Referenzkörper sind in einem Navigationssystem zur Planung und Durchführung von Operationen registriert, wobei Lagedaten des ersten Referenzkörpers und des zweiten Referenzkörpers relativ zu einem Bezugspunkt durch das Navigationssystem bestimmt werden. Zur Registrierung ist es vorteilhaft, wenn der erste Referenzkörper und der zweite Referenzkörper Lokalisatoren, wie z.B. Sensorspulen oder optische Lokalisatoren aufweisen. Die Verwendung von Sensorspulen in derartigen Navigationssystemen ist grundsätzlich bekannt. Mittels der bestimmten Lagedaten des ersten Referenzkörpers und des zweiten Referenzkörpers können die tomografischen Bilddaten und weitere Bilddaten in dem Navigationssystem auf der Anzeige überlagert dargestellt werden. Hierdurch wird eine genaue Navigation unter Zuhilfenahme intraoperativ gewonnener sowie entzerrter Röntgenbilder ermöglicht. Die Genauigkeit der Überlagerung der Bilddaten wird durch die Registrierung des ersten Referenzkörpers und des zweiten Referenzkörpers im Navigationssystem optimiert.

Die Verwendung von zwei Referenzkörpern in Kombination miteinander, von denen ein erster der Körper mit bekannter Geometrie und der zweite ein kleinerer lokaler Lokalisator am Operationsort ist erlaubt es, intraoperativ räumliche Informationen zu gewinnen und in intraoperativ erzeugte Röntgenbilder einzublenden.

Es können auch zwei der Referenzkörper Körper mit bekannter Geometrie sein. Dies ermöglicht es beispielsweise aus z.B. röntgentechnisch gewonnen Fluoroskopiebildern Rauminformationen zu gewinnen, insbesondere wenn mehrere zweidimensionale Bilder ohne Tiefeninformation (z.B. röntgentechnisch gewonnene Bilder) aus unterschiedlichen Perspektiven aufgenommen wurden und miteinander in Beziehung gesetzt werden.

Vorteilhaft ist, wenn der erste Referenzkörper und/oder der zweite Referenzkörper mittels eines elektromagnetischen und/oder optischen Lageerfassungssystems lageerfasst werden. Bevorzugt erfolgt die Lageerfassung zeitgleich zur Aufnahme der Bilddaten und/oder zeitgleich zur Aufnahme der weiteren Bilddaten.

Sowohl elektromagnetische als auch optische Lageerfassungssysteme sind grundsätzlich aus dem Stand der Technik bekannt. Bekannt sind beispielsweise elektromagnetische Lageerfassungssysteme, die einen Feldgenerator aufweisen. Der Feldgenerator erzeugt in einem Operationsgebiet ein i.d.R. alternierendes elektrisches Wechselfeld. An einem in diesem Operationsgebiet zu navigierenden medizinischen Instrument sind Lokalisierungselemente angeordnet, die Spulen aufweisen. Das elektromagnetische Wechselfeld induziert in diesen Spulen in Abhängigkeit von Ausrichtung der jeweiligen Spule zum elektromagnetischen Wechselfeld charakteristische Ströme, aus denen die Lageinformationen der Spulen bestimmbar sind.

Der erste Referenzkörper und/oder der zweite Referenzkörper kann eine Sensorspule als Lokalisator und/oder optische Lokalisatoren aufweisen, die bevorzugt am Referenzkörper befestigt oder in diesen integriert ist. Bevorzugt ist die Lage der Referenzkörper bezüglich eines Lokalisators bekannt. Der erste Referenzkörper und/oder der zweite Referenzkörper können an einem Patienten anbringbar und/oder anordenbar ausgebildet sein und so als Patientenlokalisator und/oder Patientenreferenz für eine Lageerfassung und/oder Navigation dienen.

Bevorzugt ist ein erster Referenzkörper dazu bestimmt an einem Patienten angeordnet und nach erfolgter Registrierung wieder entfernt zu werden. Bevorzugt ist ein zweiter Referenzkörper dazu bestimmt an einem Patienten angeordnet zu werden und nach erfolgter Registrierung zumindest zeitweise dort zu verbleiben. Ein erster und zweiter Referenzkörper können gleichzeitig am Patienten angeordnet sein, so dass auch ihre relative Lage zueinander durch eine Bildaufnahme aufgenommen und/oder durch ein Lageerfassungssystem bestimmt werden kann.

Zusätzlich oder alternativ kann zumindest ein Teil der tomografischen oder röntgentechnisch gewonnenen Bilddaten während einer Operation (intraoperativ) aufgenommen werden und ist somit jeweils ganz aktuell. Insbesondere bietet es sich an, intraoperativ Bilddaten mittels eines kompakten Röntgengerätes wie z.B. einem C-Bogen aufzunehmen. Häufig sind solche intraoperativ aufgenommenen Bilddaten Fluoroskopiebilder ohne Tiefeninfomation, und enthalten keine Tomografien. Mit Hilfe eines oder mehrerer Referenzkörper bekannter Geometrie können dann gleichwohl Rauminformationen gewonnen werden. Letzeres gilt vor allem - aber nicht nur - dann, wenn beispielsweise auch präoperative gewonnene Bilddaten mit Rauminformation, z.B. Tomografien wie Computer-Tomografien oder Magnet-Resonanz-Tomografien vorliegen, die mit intraoperativ gewonnen Bilddaten intraoperativ verknüpft werden.

Bezüglich des medizinischen Systems wird die Aufgabe durch ein medizinisches System zur Operationsplanung und/oder intraoperativen Navigation gelöst, welches wenigstens eine Recheneinheit, mindestens eine erste Aufnahmeeinheit zum Aufnehmen von tomografischen Bilddaten eines Körperbereichs eines Patienten, mindestens eine zweite Aufnahmeeinheit zum Aufnehmen von weiteren Bilddaten desselben Körperbereichs des Patienten sowie einen ersten Referenzkörper aufweist, wobei das medizinisches System zur Durchführung eines Verfahrens der zuvor beschriebenen Art ausgebildet ist.

Der Referenzkörper kann teilweise röntgendurchlässig sein. Bevorzugt weist der Referenzkörper röntgenundurchlässige (röntgenopake) Bestandteile, insbesondere röntgenopake Referenzmarken auf, wobei bevorzugt die geometrische Lage der Referenzmarken zueinander bekannt und auf dem Referenzkörper festgelegt ist. Auf diese Art und Weise kann ein vorbeschriebenes Vergleichen und Entzerren von aufgenommen Bilddaten anhand der geometrischen Lage der Referenzmarken zueinander erfolgen. Alternativ oder zusätzlich kann Vergleichen und Entzerren von aufgenommen Bilddaten anhand der Eigengeometrie des Referenzkörpers erfolgen.

Bevorzugt sind die Referenzmarken flächig ausgebildet und am Referenzkörper derart angeordnet, dass sich wenigstens zwei, bevorzugt wenigstens 3 Referenzmarken flächig in einer gemeinsamen Ebene erstrecken.

Eine Referenzmarke ist vorzugsweise ringförmig oder kreisförmig und/oder weist ringförmige und/oder kreisförmige Bereiche und/oder Elemente auf.

Die Aufgabe bezüglich des elektromagnetischen Feldgenerators wird durch einen elektromagnetischen Feldgenerator gelöst, der einen vorbeschriebenen Referenzkörper aufweist oder bildet. Es hat sich als vorteilhaft herausgestellt, wenn der Referenzkörper und/oder die auf dem Referenzkörper angeordneten Referenzmarken bezüglich des elektromagnetischen Feldgenerators, insbesondere bezüglich eines feldgenerierenden Elementes des Feldgenerators lagefest angeordnet sind. Bevorzugt ist die geometrische Lage der Referenzmarken zueinander bekannt und auf dem Referenzkörper festgelegt.

Der Feldgenerator und/oder das feldgenerierende Element des Feldgenerators kann rahmenförmig ausgebildet sein, insbesondere so, dass der Rahmen ein Fenster aufspannt, das - abgesehen von im Fenster anzuordnenden röntgenundurchlässigen Referenzmarkern - röntgendurchlässig ist. Das röntgendurchlässige Fenster kann durch einen flächigen röntgendurchlässigen Referenzkörper gebildet sein, auf dem röntgenundurchlässige Referenzkörper angeordnet sind. Weiter bevorzugt ist eine Oberfläche des Feldgenerator plan ausgebildet um im Betrieb auf einem Operationstisch zu liegen. Liegt während einer Operation ein Patient auf dem Operationstisch so ist der Feldgenerator bevorzugt zwischen Patient und Operationstisch angeordnet. Der Feldgenerator kann Polsterelemente aufweisen, um Druckstellen auf eine Körperoberfläche des Patienten zu vermeiden.

Die Erfindung soll nun anhand von verschiedenen Ausführungsbeispielen mit Hilfe von Figuren näher erläutert werden. In den Figuren zeigt:
Fig. 1 eine schematische Draufsicht auf eine erste Ausführungsform eines erfindungsgemäßen ersten Referenzkörpers;
Fig. 2 eine schematische Draufsicht auf eine zweite Ausführungsform eines erfindungsgemäßen ersten Referenzkörpers;
Fig. 3 eine schematische Draufsicht auf eine dritte Ausführungsform eines erfindungsgemäßen ersten Referenzkörpers;
Fig. 4 eine schematische Darstellung einer vierten Ausführungsform eines erfindungsgemäßen ersten Referenzkörpers, der Referenzmarken aufweist;

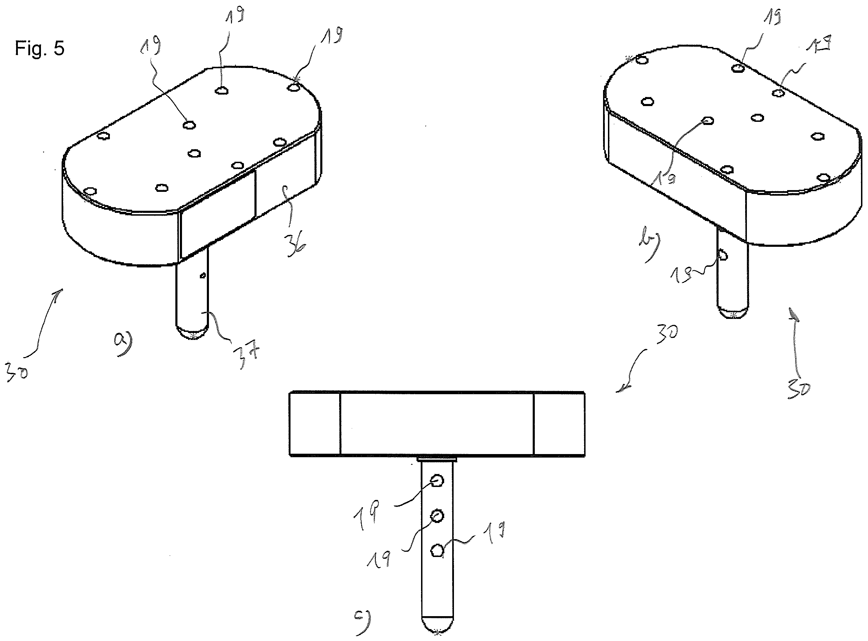

Fig. 5 eine schematische Darstellung einer fünften Ausführungsform eines erfindungsgemäßen ersten Referenzkörpers, der Referenzmarken aufweist;
Fig. 6 eine schematische Darstellung einer ersten Ausführungsform eines erfindungsgemäßen zweiten Referenzkörpers, der Referenzmarken aufweist;
Fig. 7 eine weitere schematische Darstellung eines zweiten Referenzkörpers aus Fig. 6;
Fig. 8 eine schematische Darstellung einer zweiten Ausführungsform eines erfindungsgemäßen zweiten Referenzkörpers, der Referenzmarken aufweist;
Fig. 9 eine schematische Darstellung einer Ausführungsform eines erfindungsgemäßen Feldgenerators:
Fig. 10 eine schematische Darstellung einer Anordnung zur interoperativen Bildaufnahme und
Fig. 11 eine schematische Darstellung eines erfindungsgemäßen Verfahrens zur Einbindung von Bilddaten eines Patienten in ein System zur Operationsplanung und/oder intraoperativen Navigation.

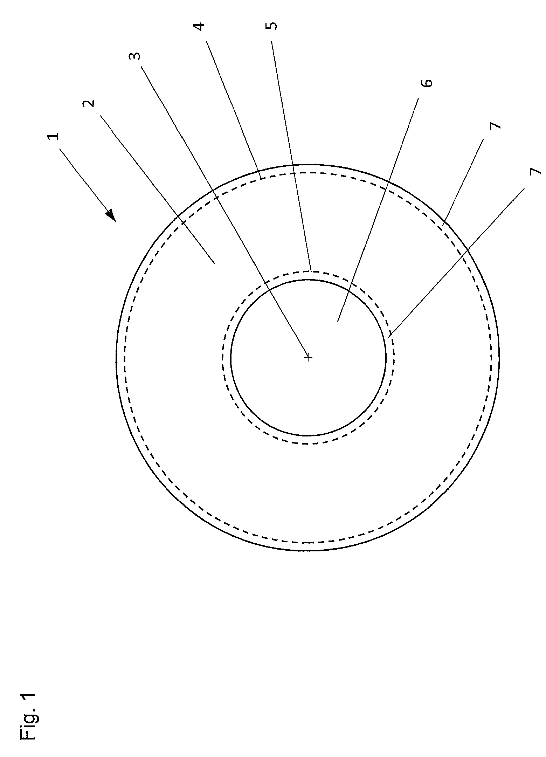

Die in Fig. 1 schematisch abgebildete erste Ausführungsform eines erfindungsgemäßen ersten Referenzkörpers 1 weist einen strichlinienförmig dargestellten, ringförmigen Grundkörper 2 mit einem Mittelpunkt 3, einem Außenumfang 4 und einem kleineren Innenumfang 5 auf. Durch den inneren Umfang 5 ist eine kreisförmige Durchführung 6 des Grundkörpers 2 begrenzt, die konzentrisch mit dem Grundkörper 2 ist. Der Grundkörper 2 ist vorzugsweise aus einem Material gebildet, das eine schlechte Durchlässigkeit von Röntgenstrahlen aufweist. Hierfür sind insbesondere Metalle, wie z.B. Blei geeignet. In dieser Ansicht ist nicht erkennbar, dass beide Oberflächen des Grundkörpers 2 flach ausgebildet sind. In alternativen Ausführungsformen können auch eine oder beide Oberflächen eine konvexe Wölbung aufweisen, die sich radial zum Mittelpunkt 3 erstreckt.

Der Grundkörper 2 ist komplett von einer Deckschicht 7 aus einem Material ummantelt, das sich vom Material des Grundkörpers 2 unterscheidet. In diesem ersten Ausführungsbeispiel ist die Deckschicht 7 genau entlang der Form des Grundkörpers 2 angeordnet. Vorzugsweise weist die Deckschicht 7 eine deutlich bessere Durchlässigkeit von Röntgenstrahlen als der Grundkörper 2 auf. Als Material der Deckschicht 7 sind insbesondere Kunststoffe geeignet. Die Deckschicht 7 ist optional und bietet z.B. hygienische Vorteile. Ferner kann eine geeignete Deckschicht 7 auch die Formstabilität des ersten Referenzkörpers 1 gewährleisten. Letzteres ist insbesondere dann wichtig, wenn der Grundkörper 2 aus einem sehr duktilen Material, wie z.B. Blei, gebildet ist. Eine glatte Oberfläche sowie hohe Sprödigkeit der Deckschicht 7 sind besonders vorteilhaft. Dabei sollte die Deckschicht eine derartige Sprödigkeit aufweisen, dass eine starke Deformation des ersten Referenzkörpers 1, die über die elastische Verformbarkeit hinausgeht, ein zumindest örtliches Brechen der Deckschicht 6 bewirkt. Somit dient eine gebrochene Deckschicht 6 als Indikator für einen deformierten ersten Referenzkörper 1, der als solcher nicht mehr verwendet werden sollte.

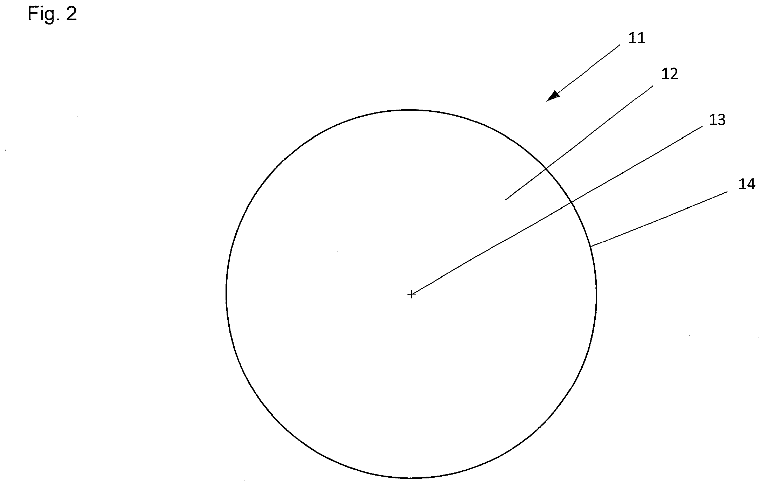

Die in Fig. 2 dargestellte zweite Ausführungsform eines erfindungsgemäßen ersten Referenzkörpers 11 weist einen kreisförmigen Grundkörper 12 mit einer Mittelpunkt 13 und einem Außenumfang 14 auf. Eine Deckschicht ist in dem abgebildeten zweiten Ausführungsbeispiel nicht vorhanden, kann aber bei Bedarf hinzugefügt werden.

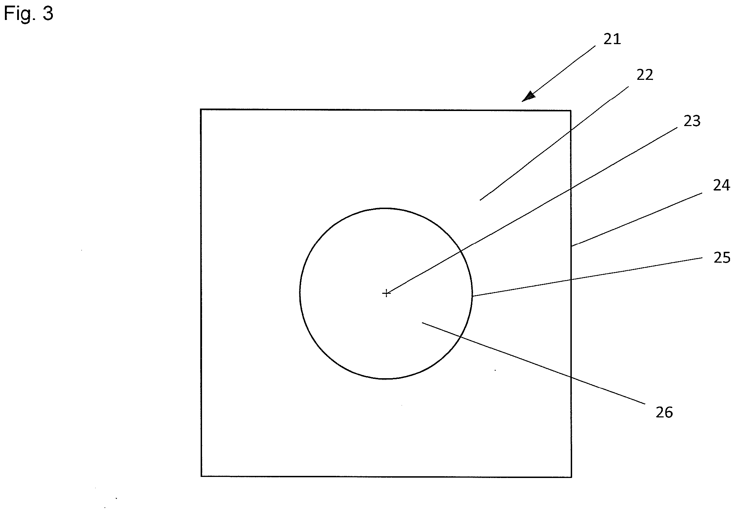

In Fig. 3 ist eine dritte Ausführungsform eines erfindungsgemäßen ersten Referenzkörpers 21 abgebildet. Der erste Referenzkörper 21 weist einen quadratischen Grundkörper 22 mit einem Mittelpunkt 23, einem Außenumfang 24 und einer kreisförmigen Durchführung 26 auf, die konzentrisch mit dem Grundkörper 22 ist. Die Durchführung 26 ist durch einen Innenumfang 25 des Grundkörpers 22 in radialer Richtung nach außen begrenzt.

Für den Fall, dass der Grundkörper 2, 12, 22 eine ausreichende Festigkeit aufweist, dass er sich unter bestimmungsgemäßen Einsatzbedingungen nicht dauerhaft verformt, kann die etwaige Deckschicht 7 aus einem elastischen Material gebildet sein, z.B. um den Grundkörper 2, 12, 22 vor äußeren Einflüssen wie z.B. Stößen zu schützen. In Frage kommen hierbei insbesondere gummielastische Werkstoffe, Silikone, etc. Bevorzugt kann die Deckschicht auch als elastische oder starre Hülle ausgebildet sein, in die der Grundkörper 2, 12, 22 einsetzbar ist.

Sofern die Deckschicht 7 eine deutlich bessere Durchlässigkeit von Röntgenstrahlen als der Grundkörper 2 aufweist, kann die Deckschicht 7 auch den Grundkörper 2, 12, 22 nebst Durchführungen 6, 26 komplett ummanteln. Auf einer Röntgenaufnahme des ersten Referenzkörpers 1, 11, 21 ist somit im Wesentlichen der Grundkörper 2, 12, 22 erkennbar.

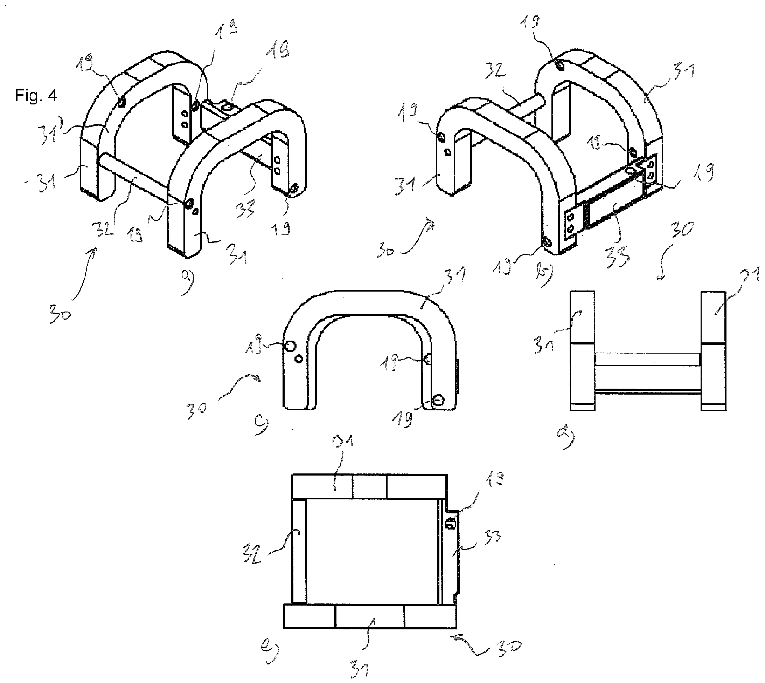

Ein in Fig. 4 dargestellter Referenzkörper 30 ist als Käfig ausgebildet und dazu bestimmt in ein offenes Operationsgebiet, beispielsweise einen frei gelegten Wirbelkörper, gestellt zu werden. Der Referenzkörper 30 weist mehrere Referenzmarken 19 auf, deren geometrische Lage zueinander bekannt und konstant ist. Der Referenzkörper 30 ist aus einem Material gebildet, das eine höhere Röntgendurchlässigkeit als die Referenzmarken 19 aufweist. Der Referenzkörper 30 weist zwei u-förmige Abschnitte 31 auf, die zueinander fluchtend angeordnet und über zwei Stege 32, 33 miteinander verbunden sind. Auf einer Oberfläche 31' des Referenzkörper 30 sind zwei flächige Referenzmarken 19 derart angeordnet, dass sie sich flächig in einer gemeinsamen Ebene, nämlich der Oberfläche 31'erstrecken. Die Unterfiguren a) bis e) zeigen jeweils unterschiedliche Ansichten den gleichen Referenzkörpers 30.

Ein in Fig, 5 dargestellter alternativer Referenzkörper 30 ist pilzförmig ausgebildet und dazu bestimmt in eine chirurgische Hautöffnung gesteckt zu werden, die beispielsweise mittels eines Trokars im Rahmen eines minimal invasiven Eingriffs eröffnet wurde. Der Referenzkörper 30 weist eine flache Kappe 36 auf an der zentral ein Stift 37 angebracht ist. Der Referenzkörper 30 weist sowohl auf der Kappe 36 als auch auf dem Stift 37 mehrere Referenzmarken 19 auf, deren geometrische Lage zueinander bekannt und konstant ist. Der Referenzkörper 30 ist aus einem Material gebildet, dass eine höhere Röntgendurchlässigkeit als die Referenzmarken 19 aufweist. Die Unterfiguren a) bis c) zeigen jeweils unterschiedliche Ansichten den gleichen Referenzkörpers 30.

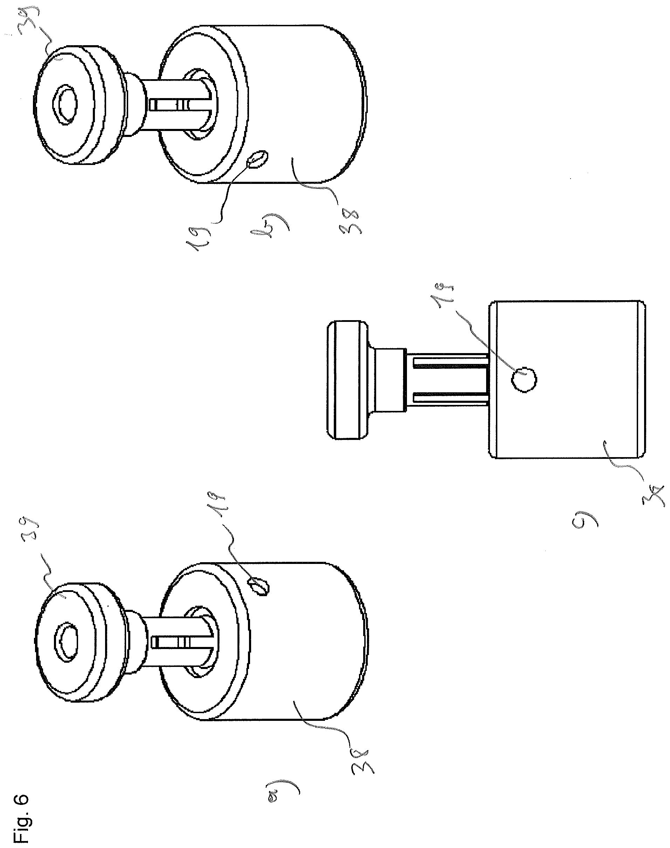

Ein Fig. 6 dargestellter anderer alternativer Referenzkörper 30 ist hülsenförmig ausgebildet und dazu bestimmt beispielsweise auf einem chirurgischen Draht, beispielsweise einen am Knochen zu befestigenden Kirschner Draht aufgezogen und fixiert zu werden. Der Referenzkörper weist dazu eine Außenhülse 38 und eine einschiebbare Fixierhülse 39 auf. Der Referenzkörpers 30 weist auf der Außenhülse 38 mehrere Referenzmarken 19 auf, deren geometrische Lage zueinander bekannt und konstant ist. Die Referenzmarken 19 sind gleichmäßig in Umfangsrichtung an der Außenhülse 38 angeordnet. Der Referenzkörper 30 ist aus einem Material gebildet, dass eine höhere Röntgendurchlässigkeit als die Referenzmarken 19 aufweist. Die Unterfiguren a) bis c) zeigen jeweils unterschiedliche Ansichten den gleichen Referenzkörpers 30.

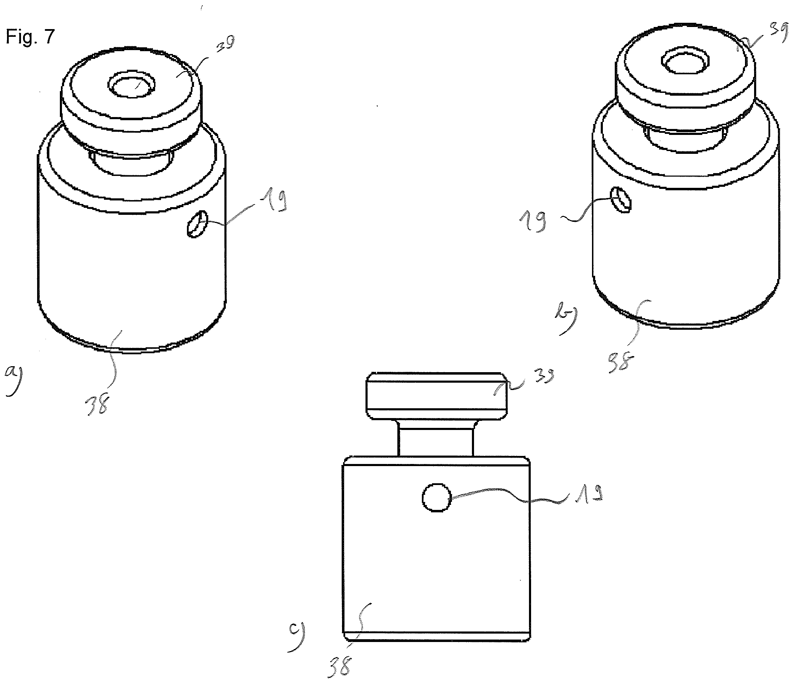

Ein in Fig. 7 zeigt den Referenzkörpers 30 aus Fig, 6 in Fixierstellung, d.h. der Referenzkörper ist auf einem chirurgischen Draht (nicht gezeigt) fixiert. Dazu ist die Fixierhülse 39 in die Außenhülse 38 hineingedrückt. Die Unterfiguren a) bis c) zeigen jeweils unterschiedliche Ansichten den gleichen Referenzkörpers 30.

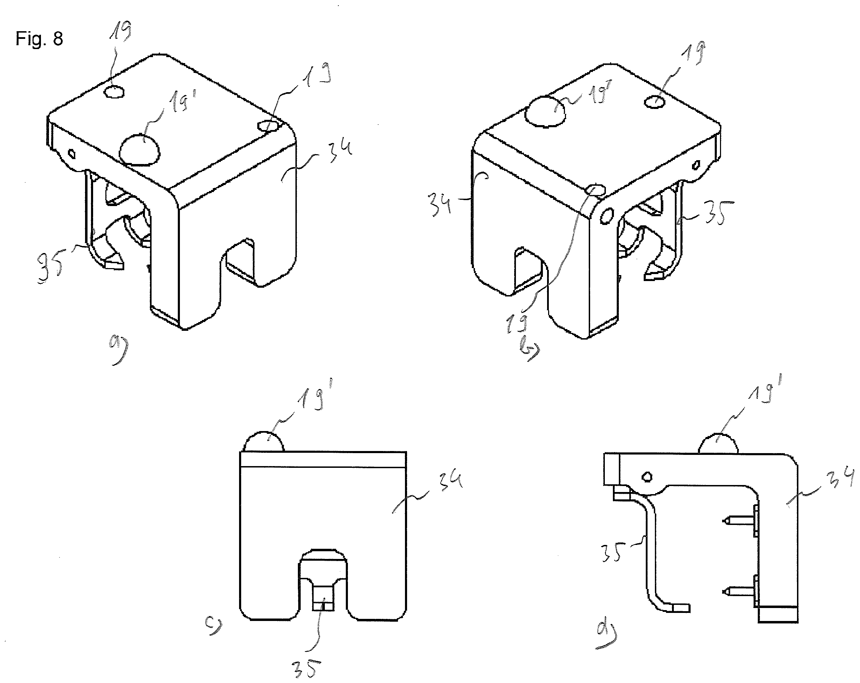

Ein weiterer, alternativer in Fig. 8 dargestellter Referenzkörper 30 ist klemmenförmig ausgebildet und dazu bestimmt beispielsweise an einem Wirbel fixiert zu werden. Dazu weist der Referenzkörper 30 einen winkelförmigen Hauptbügel 34 auf an dem schwenkbar ein Klemmbügel 35 angebracht ist. Der Referenzkörper 30 weist mehrere Referenzmarken 19, 19' auf, deren geometrische Lage zueinander bekannt und konstant ist. Vorliegend sind die Referenzmarken 19, 19' nur auf einer Oberfläche des winkelförmigen Hauptbügels 34 angeordnet und weisen unterschiedliche Größen auf. Der Referenzkörper 30 ist aus einem Material gebildet, dass eine höhere Röntgendurchlässigkeit als die Referenzmarken 19 aufweist. Die Unterfiguren a) bis d) zeigen jeweils unterschiedliche Ansichten den gleichen Referenzkörpers 30.

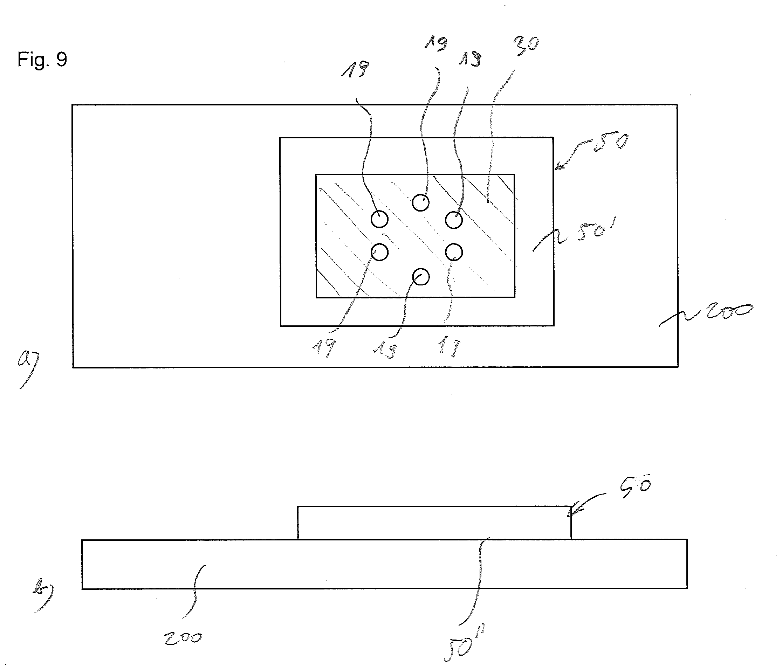

Fig. 9 a) zeigt eine Draufsicht auf einen Operationstisch 200, wobei ein erfindungsgemäßer Feldgenerator 50 auf dem Operationstisch 200 aufliegt. Der Feldgenerator 50 und das (integrierte und daher nicht gezeigte) feldgenerierende Element des Feldgenerators 50 sind rahmenförmig ausgebildet. Der Rahmen 50' des Feldgenerators 50 spannt ein Fenster auf (schraffierte Fläche), das röntgendurchlässig ist. Das röntgendurchlässige Fenster ist vorliegend durch einen flächigen röntgendurchlässigen Referenzkörper 30 gebildet. Auf dem röntgendurchlässigen Referenzkörper 30 sind Referenzmarken 19 angeordnet, die jeweils lagefest bezüglich des Feldgenerators 50 und des integrierten und daher nicht gezeigten feldgenerierenden Elements angeordnet sind. Die geometrisehe Lage der röntgenundurchlässigen Referenzmarken 19 zueinander ist bekannt und konstant.

Wie aus Seitenansicht Fig. 9b) ersichtlich ist, ist der Feldgenerator 50 mit einer planen Oberfläche 50" ausgebildet und liegt auf dem Operationstisch 200 auf.

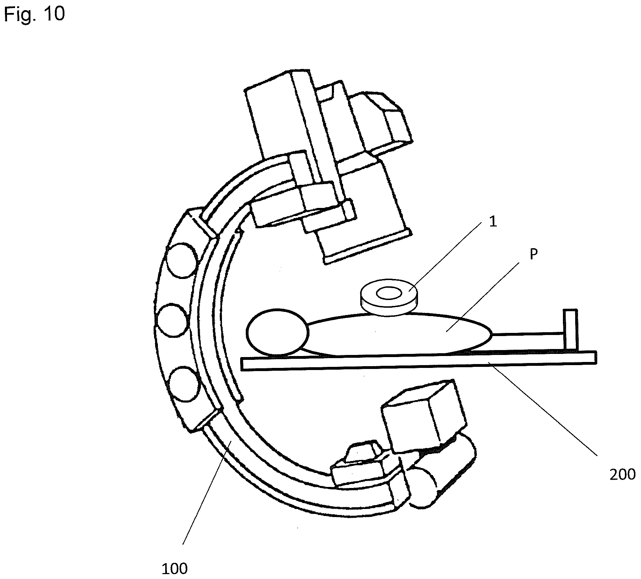

Einer Anordnung zur interoperativen Bildaufnahme ist in Fig. 10 gezeigt. Ein erster Referenzkörpers 1, beispielhaft der mit Bezug auf Fig. 1 beschriebene, ist abdominal an einem auf einem Operationstisch 200 liegenden Patienten P angeordnet. Der Patent P und der Referenzkörper 1 sind derart in einem C-Bogen positioniert, dass die röntgentechnisch gewonnenen Bilddaten sowohl einen definierten Körperbereichs des Patienten P, vorliegend das Abdomen, als auch den dort Angeordneten Referenzkörper 1 umfassen.

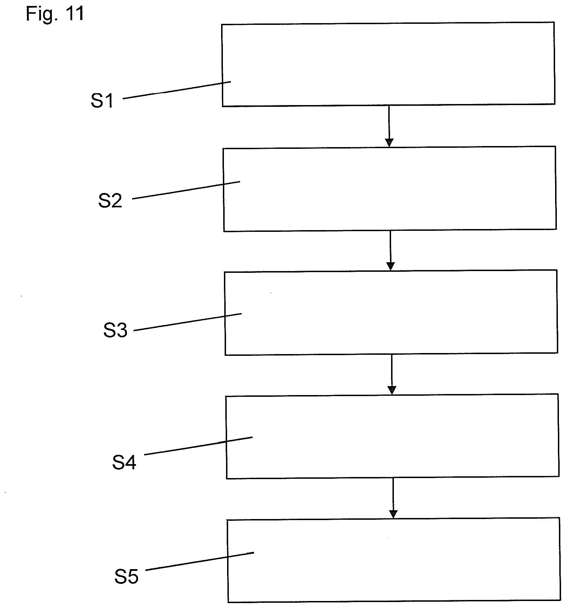

Bei einem erfindungsgemäßen Verfahren zur Einbindung von Bilddaten eines Patienten in ein System zur Operationsplanung und/oder intraoperativen Navigation in Fig. 11 werden in einem ersten Schritt S1 tomografischen Bilddaten oder röntgentechnisch gewonnenen Bilddaten von zumindest einem definierten Körperbereich des Patienten mittels mindestens einem hierfür geeigneten ersten Aufnahmegerät aufgenommen, wobei ein erster Referenzkörper mit mindestens einer Oberfläche an dem Patienten angeordnet ist und von dem ersten Aufnahmegerät mit aufgenommen wird.

In einem zweiten Schritt S2 werden die den ersten Referenzkörper repräsentierenden aufgenommenen Bilddaten mit bekannten geometrischen Daten des ersten Referenzkörpers zum Gewinnen von Verzerrungsinformation verglichen.

In einem dritten Schritt S3 werden aufgenommene Bilddaten durch eine Recheneinheit auf Basis der Verzerrungsinformation zum Gewinnen entzerrter Bilddaten entzerrt.

In einem vierten Schritt S4 werden die entzerrten Bilddaten mit weiteren Bilddaten desselben Körperbereichs des Patienten zum Gewinnen überlagerter Bilddaten überlagert.

In einem fünften Schritt S5 werden die überlagerten Bilddaten auf einer Anzeige dargestellt.

### Bezugszeichenliste

- 1, 11, 21, 30: Referenzkörper
- 2, 12, 22: Grundkörper
- 3, 13, 23: Mittelpunkt
- 4, 14, 24: Außenumfang
- 5, 25: Innenumfang
- 6, 26: kreisförmige Durchführung
- 7: Deckschicht
- 19, 19': Referenzmarke
- 31: U-förmiger Abschnitt
- 31': Oberfläche des Referenzkörpers
- 32, 33: Steg
- 34: Hauptbügel
- 35: Klemmbügel
- 36: Kappe
- 37: Stift
- 38: Außenhülse
- 39: Fixierhülse
- 50: Feldgenerator
- 50': Rahmen des Feldgenerators
- 50": plane Oberfläche des Feldgenerators
- 100: C-Bogen
- 200: Operationstisch
- P: Patient
- S1, S2, S3, S4, S5: Verfahrensschritte

## Patentansprüche

1. Verfahren zum Betreiben eines medizinischen Systems zur Operationsplanung und/oder intraoperativen Navigation zum Darstellen (S5) durch eine Recheneinheit empfangener (S1), verglichener (S2), entzerrter (S3) und überlagerter (S4) Bilddaten auf einer Anzeige, **gekennzeichnet durch** die Schritte:
- Bereitstellen eines ersten Referenzkörpers (1, 11, 21) mit bekannter Geometrie;
- Bereitstellen von geometrischen Daten des ersten Referenzkörpers;
- Anordnen des ersten Referenzkörpers (1, 11, 21) mit mindestens einer Oberfläche an einem Patienten;
- Aufnehmen (S1) von tomografischen Bilddaten oder röntgentechnisch gewonnenen Bilddaten von zumindest einem definierten Körperbereich des Patienten mittels mindestens einem hierfür geeigneten ersten Aufnahmegerät, wobei der erste Referenzkörper (1, 11, 21) von dem ersten Aufnahmegerät mit aufgenommen wird;
- Vergleichen (S2) der den ersten Referenzkörper repräsentierenden aufgenommenen Bilddaten mit den bekannten geometrischen Daten des ersten Referenzkörpers zum Gewinnen von Verzerrungsinformation;
- Entzerren (S3) der aufgenommenen Bilddaten durch eine Recheneinheit auf Basis der Verzerrungsinformation zum Gewinnen entzerrter Bilddaten;
- Überlagern (S4) der entzerrten Bilddaten mit weiteren, mittels einem zweiten Aufnahmegerät aufgenommenen Bilddaten desselben Körperbereichs des Patienten zum Gewinnen überlagerter Bilddaten, wobei auch die weiteren Bilddaten den ersten Referenzkörper bekannter geometrischer Daten umfassen und ebenfalls durch Vergleich mit diesen bekannten Daten vor dem Überlagern durch die Recheneinheit entzerrt wurden; und
- Darstellen (S5) der überlagerten Bilddaten auf einer Anzeige.

2. Verfahren nach Anspruch 1,
**dadurch gekennzeichnet, dass** die geometrischen Daten des ersten Referenzkörpers (1, 11, 21) Bilddaten sind, die die tatsächliche Geometrie des ersten Referenzkörpers (1, 11, 21) unverzerrt abbilden, wobei der erste Referenzkörper (1, 11, 21) vorzugsweise ringförmig oder kreisförmig ist und/oder ringförmige und/oder kreisförmige Bereiche und/oder Elemente aufweist.

3. Verfahren nach Anspruch 2,
**dadurch gekennzeichnet, dass** der erste Referenzkörper (1, 11, 21) bei der Aufnahme der weiteren Bilddaten die gleiche Lage zum Aufnahmegerät und/oder Patienten aufweist wie bei der Aufnahme der tomografischen Bilddaten.

4. Verfahren nach Anspruch 2 oder 3,
**dadurch gekennzeichnet, dass** der erste Referenzkörper (1, 11, 21) bei der Aufnahme der weiteren Bilddaten den gleichen Abstand zum Aufnahmegerät aufweist, wie bei der Aufnahme der tomografischen Bilddaten.

5. Verfahren nach einem der vorangehenden Ansprüche,
**dadurch gekennzeichnet, dass** der erste Referenzkörper (1, 11, 21) während des Aufnehmens der tomografischen Bilddaten derart ausgerichtet ist, dass der größtmögliche Teil der Oberfläche des ersten Referenzkörpers (1, 11, 21) dem Aufnahmegerät zugewandt ist, wobei der erste Referenzkörper (1, 11, 21) vorzugsweise an einer Körperstelle des Patienten angeordnet wird, und/oder dass der definierte Körperbereich des Patienten vorzugsweise die Wirbelsäule umfasst.

6. Verfahren nach einem der vorangehenden Ansprüche,
**dadurch gekennzeichnet, dass** die weiteren Bilddaten desselben Körperbereichs des Patienten von einem zweiten Aufnahmegerät aufgenommen werden, wobei das zweite Aufnahmegerät ein Röntgengerät ist und die weiteren Bilddaten Fluoroskopiebilder umfassen.

7. Verfahren nach einem der vorangehenden Ansprüche,
**dadurch gekennzeichnet, dass** die weiteren Bilddaten intraoperativ aufgenommen werden und/oder dass zumindest ein Teil der tomografischen Bilddaten während einer Untersuchung oder Operation aufgenommen wird.

8. Verfahren nach einem der vorangehenden Ansprüche,
**dadurch gekennzeichnet, dass** ein zweiter Referenzkörper an einem Operationsort im Körper des Patienten angeordnet ist, wobei der Operationsbereich im definierten Körperbereich des Patienten angeordnet ist, wobei der erste Referenzkörper und der zweite Referenzkörper in einem Navigationssystem zur Planung und Durchführung von Operationen registriert sind, wobei Lagedaten des ersten Referenzkörpers und des zweiten Referenzkörpers relativ zu einem Bezugspunkt durch das Navigationssystem bestimmt werden, wobei die tomografischen Bilddaten und weiteren Bilddaten mittels der Lagedaten des ersten Referenzkörpers und des zweiten Referenzkörpers in dem Navigationssystem auf der Anzeige überlagert dargestellt werden.

9. Verfahren nach einem der vorangehenden Ansprüche, **dadurch gekennzeichnet, dass** der erste Referenzkörper mittels eines elektromagnetischen und/oder optischen Lageerfassungssystems lageerfasst wird, wobei vorzugsweise die Lageerfassung zeitgleich zur Aufnahme der Bilddaten und/oder zeitgleich zur Aufnahme der weiteren Bilddaten erfolgt.

10. Verfahren nach Anspruch 8, **dadurch gekennzeichnet, dass** der zweite Referenzkörper mittels eines elektromagnetischen und/oder optischen Lageerfassungssystems lageerfasst wird, wobei vorzugsweise die Lageerfassung zeitgleich zur Aufnahme der Bilddaten und/oder zeitgleich zur Aufnahme der weiteren Bilddaten erfolgt.

11. Verfahren nach einem der Ansprüche 1 bis 7 oder 9, **dadurch gekennzeichnet, dass** der erste Referenzkörper (1, 11, 21, 30) Referenzmarken (19) aufweist, deren geometrische Lage zueinander bekannt und konstant ist.

12. Verfahren nach einem der Ansprüche 8 oder 10, **dadurch gekennzeichnet, dass** der zweite Referenzkörper Referenzmarken (19) aufweist, deren geometrische Lage zueinander bekannt und konstant ist.

13. Verfahren nach Anspruch 11, **dadurch gekennzeichnet, dass** der erste Referenzkörper (30) an einem Feldgenerator (50) angeordnet ist, wobei insbesondere der Feldgenerator (50) und/oder das feldgenerierende Elemente des Feldgenerators (50) rahmenförmig ausgebildet sind, insbesondere so, dass der Rahmen (50') ein Fenster aufspannt, das - abgesehen von im Fenster anzuordnenden röntgenundurchlässigen Referenzmarken (19) - röntgendurchlässig ist.

14. Medizinisches System zur Operationsplanung und/oder intraoperativen Navigation, mit einer Recheneinheit, mindestens einer ersten Aufnahmeeinheit zum Aufnehmen von tomografischen Bilddaten eines Körperbereichs eines Patienten, mindestens einer zweiten Aufnahmeeinheit zum Aufnehmen von weiteren Bilddaten desselben Körperbereichs des Patienten sowie einem Referenzkörper, wobei das medizinische System mit einem Verfahren nach einem der Ansprüche 1 bis 7 oder 9 oder 11 oder 13 betrieben wird.

## Claims

1. Method of operating a medical system for surgical planning and/or intrasurgical navigation for displaying (S5), on a display, image data received (S1), compared (S2), rectified (S3) and superimposed (S4) by a computing unit, marked by the following steps:
- providing a first reference body (1, 11, 21) with known geometry;
- providing geometric data of the first reference body;
- arranging the first reference body (1, 11, 21) with at least one surface on a patient;
- recording (S1) of tomographic image data or x-ray obtained image data of at least one defined body area of the patient by means of at least one suitable first recording device, wherein the first reference body (1, 11, 21) from the first recording device is included;
- comparing (S2) the recorded image data representing the first reference body with the known geometric data of the first reference body to obtain distortion information;
- rectifying (S3) the recorded image data by means of a computing unit based on the distortion information to obtain the rectified image data;
- superimposing (S4) the rectified image data with further image data of the same body area of the patient, recorded by means of a second recording device, to obtain superimposed image data, wherein the further image data also comprises the first reference body of known geometric data and has also been rectified by means of comparison with this known data by the computing unit prior to superimposing; and
- displaying (S5) the superimposed image data on a display.

2. Method according to claim 1,
**characterized in that** the geometric data of the first reference body (1, 11, 21) represents image data that shows an undistorted display of the actual geometry of the first reference body (1, 11, 21), wherein the first reference body (1, 11, 21) is preferably annular or circular and/or has annular and/or circular areas and/or elements.

3. Method according to claim 2,
**characterized in that**, when further image data is recorded, the first reference body (1, 11, 21) has the same position relative to the recording device and/or patient as during the recording of the tomographic image data.

4. Method according to claim 2 or 3,
**characterized in that**, when further image data is recorded, the first reference body (1, 11, 21) is at the same distance to the recording device as during the recording of the tomographic image data.

5. Method according to one of the above claims,
**characterized in that** the first reference body (1, 11, 21) is aligned in such a way during the recording of the tomographic image data that the largest possible part of the surface of the first reference body (1, 11, 21) is facing the recording device, wherein the first reference body (1, 11, 21) is preferably arranged on a body part of the patient, and/or that 15 the defined body area of the patient preferably includes the spinal column.

6. Method according to one of the above claims,
**characterized in that** the further image data of the same body area of the patient is recorded by a second recording device, wherein the second recording device is an x-ray device and the further image data includes fluoroscopic images.

7. Method according to one of the above claims,
**characterized in that** the further image data is recorded intrasurgically, and/or that at least part of the tomographic image data is recorded during an examination or operation.

8. Method according to one of the above claims,
**characterized in that** a second reference body is arranged at a surgical site in the patient's body, wherein the surgical site is arranged in the defined body area of the patient, wherein the first reference body and the second reference body are registered in a navigation system for planning and performing surgery, wherein the position data of the first reference body and the second reference body relative to a reference point are determined by the navigation system, wherein the tomographic image data and further image data are shown superimposed on the display by means of the position data of the first reference body and the second reference body in the navigation system.

9. Method according to one of the above claims, **characterized in that**
the position of the first reference body is detected by means of an electromagnetic and/or optical position detection system, wherein the position detection preferably takes place simultaneously with the recording of the image data and/or simultaneously with the recording of the further image data.

10. Method according to claim 8, **characterized in that** the position of the second reference body is detected by means of an electromagnetic and/or optical position detection system, wherein the position detection preferably takes place simultaneously with the recording of the image data and/or simultaneously with the recording of the further image data.

11. Method according to one of claims 1 to 7 or 9, **characterized in that** the first reference body (1, 11, 21, 30) has reference marks (19) whose geometric positions relative to one another are known and constant.

12. Method according to one of claims 8 or 10, **characterized in that** the second reference body has reference marks (19) whose geometric positions relative to one another are known and constant.

13. Method according to claim 11, **characterized in that** the first reference body (30) is arranged on a field generator (50), wherein in particular the field generator (50) and/or the field-generating element of the field generator (50) is designed in the shape of a frame, in particular in such a way that the frame (50') spans a window which - apart from radiodense reference marks (19) to be arranged in the window - is radiolucent.

14. Medical system for surgical planning and/or intrasurgical navigation, comprising a computing unit, at least one first recording unit for recording tomographic image data of a body area of a patient, at least a second recording unit for recording further image data of the same body area of the patient, and a reference body, wherein the medical system is operated using a method according to one of claims 1 to 7 or 9 or 11 or 13.

## Revendications

1. Procédé pour faire fonctionner un système médical de planification d'intervention et/ou de navigation peropératoire pour la représentation (S5) sur un affichage de données d'image reçues (S1), comparées (S2), corrigées (S3) et superposées (S4) par une unité informatique, **caractérisé par** les stades :
- on dispose d'un premier corps (1, 11, 21) de référence de géométrie connue ;
- on dispose de données géométriques du premier corps de référence ;
- on met le premier corps (1, 11 , 21) de référence par au moins une surface sur un patient ;
- on enregistre (S1) des données d'image de tomodensitométrie ou des données d'image obtenues par la technique des rayons X d'au moins une partie définie du corps du patient, au moyen d'au moins un premier appareil d'enregistrement propre à cet effet, le premier corps (1, 11, 21) de référence étant enregistré par le premier appareil d'enregistrement ;
- on compare (S2) les données d'image enregistrées représentant le premier corps de référence aux données géométriques connues du premier corps de référence, pour obtenir de l'information de déformation ;
- on corrige (S3) les données d'image enregistrées par une unité informatique, sur la base de l'information de déformation pour obtenir des données d'image corrigées ;
- on superpose (S4) les données d'image corrigées à d'autres données d'image, enregistrées au moyen d'un deuxième appareil d'enregistrement, de la même partie du corps du patient pour obtenir des données d'image superposées, les autres données d'image du premier corps de référence comprenant également des données géométriques connues et ayant été également corrigées par l'unité informatique avant la superposition par comparaison à ces données connues ; et
- on représente (S5) les données d'image superposées sur un affichage.

2. Procédé suivant la revendication 1,
**caractérisé en ce que** les données géométriques du premier corps (1, 11, 21) de référence sont des données d'image, qui reproduisent sans déformation la géométrie réelle du premier corps (1, 11, 21) de référence, le premier corps (1, 11, 21) de référence étant de préférence annulaire ou circulaire et/ou ayant des parties et/ou des éléments annulaires et/ou circulaires.

3. Procédé suivant la revendication 2,
**caractérisé en ce que** le premier corps (1, 11, 21) de référence a, lors de l'enregistrement des autres données d'image, la même position par rapport à l'appareil d'enregistrement et/ou au patient que lors de l'enregistrement des données d'image de tomodensitométrie.

4. Procédé suivant la revendication 2 ou 3,
**caractérisé en ce que** le premier corps (1, 11, 21) de référence a, lors de l'enregistrement des autres données d'image, la même distance à l'appareil d'enregistrement que lors de l'enregistrement des données de tomodensitométrie.

5. Procédé suivant l'une des revendications précédentes, **caractérisé en ce que** le premier corps (1, 11, 21) de référence est, pendant l'enregistrement des données d'image de tomodensitométrie, orienté de manière à ce que la plus grande partie possible de la surface du premier corps (1, 11, 21) de référence soit tournée vers l'appareil d'enregistrement, le premier corps (1, 11, 21) de référence étant disposé de préférence sur un emplacement du corps du patient et/ou **en ce que** la partie définie du corps du patient comprend de préférence la colonne vertébrale.

6. Procédé suivant l'une des revendications précédentes, **caractérisé en ce que** l'on enregistre les autres données d'image de la même partie du corps du patient par un deuxième appareil d'enregistrement, le deuxième appareil d'enregistrement étant un appareil à rayons X et les autres données d'image comprenant des images de radioscopie.

7. Procédé suivant l'une des revendications précédentes, **caractérisé en ce que** l'on enregistre peropératoirement les autres données d'image et/ou **en ce que** l'on enregistre au moins une partie des données d'image de tomodensitométrie pendant un examen ou une intervention.

8. Procédé suivant l'une des revendications précédentes, **caractérisé en ce que** l'on met un deuxième corps de référence à un emplacement d'intervention du corps du patient, dans lequel la partie d'intervention est disposée dans une partie définie du corps du patient, dans lequel le premier corps de référence et le deuxième corps de référence sont enregistrés dans un système de navigation, pour planifier et effectuer des interventions, dans lequel on détermine par le système de navigation des données de position du premier corps et du deuxième corps de référence par rapport à un point de repère, dans lequel on représente sur l'affichage les données d'image de tomodensitométrie et d'autres données d'image au moyen des données de position du premier corps de référence et du deuxième corps de référence dans le système de navigation.

9. Procédé suivant l'une des revendications précédentes, **caractérisé en ce que** l'on détecte en position le premier corps de référence au moyen d'un système électromagnétique et/ou optique de détection de position, la détection de position s'effectuant de préférence en même temps que l'enregistrement des données d'image et/ou en même temps que l'enregistrement des autres données d'image.

10. Procédé suivant la revendication 8,
**caractérisé en ce que** l'on détecte en position le deuxième corps de référence au moyen d'un système électromagnétique et/ou optique de détection de position, la détection de position s'effectuant de préférence en même temps que l'enregistrement des données d'image et/ou en même temps que l'enregistrement des autres données d'image.

11. Procédé suivant l'une des revendications 1 à 7 ou 9, **caractérisé en ce que** le premier corps (1, 11, 21, 30) de référence a des marques (19) de repérage, dont la position géométrique les unes par rapport aux autres est connue et est constante.

12. Procédé suivant l'une des revendications 8 ou 10, caractérisé en ce le deuxième corps de référence a des marques (19) de repérage, dont la position géométrique les unes par rapport aux autres est connue et est constante.

13. Procédé suivant la revendication 11,
caractérisé en ce le premier corps (30) de référence est disposé sur un générateur (50) de champ, dans lequel en particulier le générateur (50) de champ et/ou des éléments produisant le champ du générateur (50) de champ sont constitués sous la forme d'un cadre, notamment de manière à ce que le cadre (50) délimite une fenêtre, qui - abstraction faite de marques (19) de repérage imperméables aux rayon X mises dans la fenêtre - est perméable aux rayons X.

14. Système médical de planification d'intervention et/ou de navigation peropératoire, comprenant une unité informatique, au moins une première unité d'enregistrement pour l'enregistrement de données d'image de tomodensitométrie d'une partie du corps d'un patient, au moins une deuxième unité d'enregistrement pour l'enregistrement d'autres données d'image de la même partie du corps du patient, ainsi qu'un corps de référence, dans lequel on fait fonctionner le système médical par un procédé suivant l'une des revendications 1 à 7 ou 9 ou 11 ou 13.
